# EUROPÄISCHE PATENTSCHRIFT

(11) **EP 2 295 102 B1**
(45) Veröffentlichungstag und Bekanntmachung des Hinweises auf die Patenterteilung: **19.10.2016**
(21) Anmeldenummer: 10180729.5
(22) Anmeldetag: 09.07.2004
(51) Int. Cl.: A61M 16/06, A61M 16/20

(54) **Atemmaskenanordnung mit einer Atemgasableitungseinrichtung**
Breathing mask assembly with a breathing gas discharge device
Agencement de masque respiratoire avec un dispositif de déviation de gaz respiratoire

(30) Priorität: 09.07.2003 DE 10331134; 30.07.2003 DE 10335162
(43) Veröffentlichungstag der Anmeldung: 16.03.2011
(62) Teilanmeldung aus: 04740878.6
(73) Patentinhaber: ResMed R&D Germany GmbH, 82152 Martinsried (DE)
(72) Erfinder: Madaus, Stefan, 82166 Gräfelfing (DE); Stauffenberg, Caspar Graf, 82131 Gauting (DE); Vögele, Harald, 82131 Gauting (DE)
(74) Vertreter: Vossius & Partner Patentanwälte Rechtsanwälte mbB

(56) Entgegenhaltungen:
- EP-A1- 0 267 428
- WO-A1-00/78381
- WO-A2-02/32491
- DE-T2- 69 328 158
- US-A- 5 465 712
- US-A1- 2001 032 648
- US-A1- 2003 075 180

## Beschreibung

### Gebiet der Erfindung

Die Erfindung betrifft eine Atemmaskenanordnung wie sie beispielsweise im Rahmen einer CPAP-Therapie zur Behandlung schlafbezogener Atmungsstörungen Anwendung finden kann. Weiterhin betrifft die Erfindung auch eine Atemgasableitungseinrichtung für eine Atemmaskenanordnung, zur Ableitung von CO2 befrachtetem Atemgas.

### Hintergrund der Erfindung

Im Rahmen der genannten CPAP-Therapie kann einem Patienten über eine Atemmaskenanordnung ein atembares Gas, insbesondere Umgebungsluft auf einem Druckniveau zugeführt werden, das über dem Umgebungsdruckniveau liegt. Durch das unter Druck stehende Atemgas kann eine pneumatische Schienung der oberen Atemwege erreicht werden und hierdurch etwaigen Obstruktionen vorgebeugt werden. Im Rahmen der Durchführung einer Druckbeatmungs- bzw. CPAP-Therapie werden die zur Zufuhr des Atemgases erforderlichen Atemmaskenanordnungen üblicherweise über die gesamte Schlaf- oder Ruhephase des Patienten hinweg von diesem getragen. Die Atemmaskenanordnung stützt sich üblicherweise über eine Dichtlippenzone im Umgebungsbereich der Nase des Maskenanwenders sowie über eine Stirnauflageeinrichtung im Stirnbereich des Maskenanwenders ab. Die zur Applikation der Atemmaskenanordnung erforderlichen Haltekräfte können durch eine Fixiereinrichtung, die zum Beispiel ein um den Hinterkopf des Maskenanwenders herumgeführtes Kopfband aufweist, aufgebracht werden. Die Ableitung des ggf. mit CO2 befrachteten Atemgases aus dem Innenbereich der Atemmaske kann über Bohrungen erfolgen, deren Durchgangsquerschnitt derart festgelegt ist, dass ein hinreichend großer Abstrom des Atemsases ermöglicht ist.

Dokument WO 02/32491 A2 offenbart eine Atemmaske zur Zufuhr eines Atemgases zu einem Maskenanwender sowie eine Ableitungseinrichtung zur Ableitung von Atemgas aus einem von einer Atemmaske definierten Maskeninnenbereich. Die Atemmaske umfasst eine Dichtlippeneinrichtung zur Abdichtung einer Maskenauflagezone, einen Maskenbasiskörper zur Bildung des Maskeninnenraumes sowie eine Anschlusseinrichtung zum Anschluss wenigstens einer Atemgasleitung, wobei der Maskenbasiskörper und/oder die Anschlusseinrichtung mit wenigstens einer aus einem Elastomermaterial gebildeten Entkoppelungsstruktur versehen ist.

Dokument US 5,465,712 offenbart eine Reanimationsmaske zur künstlichen Beatmung eines Patienten. Die Reanimationsmaske umfasst einen becherartigen Bereich, der von einer Dichtlippe umgeben ist. Ferner umfasst die Animationsmaske ein Mundstück, welches sich an den becherartigen Bereich anschließt und welches zur Wiederbelebung eines Patienten genutzt wird. Zwischen Mundstück und becherartigem Bereich ist ferner eine Ventileinheit angeordnet.

Dokument WO 00/78381 A1 offenbart eine Atemmaske mit einer Passage zum Auswaschen von ausgeatmeten Gas, die von der Maskenschale und einem an die Maskenschale angebrachten Aufsatzelement begrenzt ist.

Dokument US 2001/032648 A1 offenbart einen Ausatemluftpfad für eine CPAP-Maske, wobei ein Ausatemrohr integral mit der Maske und einem Einatemschlauch aufgeführt ist.

Dokument EP 0267428 A1 offenbart eine Atemmaske, die ein Ausatemventil als Einsatz enthält.

Dokument US 2003/075180 A1 offenbart eine Atemmaskenanordnung, wobei ein Ausatemluftpfad von einer integral mit einem Lufteinlassrohr aufgeführten Prallplatte und einem der Maskenschale zugeordneten Ausatemrohr begrenzt ist.

### Aufgabe der Erfindung

Der Erfindung liegt die Aufgabe zugrunde, eine Atemmaskenanordnung für die CPAP-Therapie bereitzustellen, die hinsichtlich Anwenderkomfort und Anwendungseffektivität verbessert ist.

### Erfindungsgemäße Lösung

Hierin wird beschrieben eine Atemmaskenanordnung mit einer Dichtlippeneinrichtung zur Auflage auf der Gesichtsfläche eines Maskenanwenders, einer Abdeckeinrichtung die im Zusammenspiel mit der Dichtlippeneinrichtung einen Maskeninnenraum definiert, und einer Atemgasleitungseinrichtung zur Zuleitung von Atemgas zu jenem von der Abdeckeinrichtung definierten und mit der Nasen- und/oder Mundöffnung des Maskenanwenders in Verbindung stehendem Maskeninnenraum, wobei die Abdeckeinrichtung zumindest abschnittsweise als luftdurchlässige Struktur ausgebildet ist.

Dadurch wird es auf vorteilhafte Weise möglich, eine Atemmaskenanordnung zu schaffen, die eine Gasabströmfläche bereitstellt welche eine diffuse Gasabströmung bei geringer Geräuschemission ermöglicht.

Vorzugsweise ist die Abdeckeinrichtung aus einem luftdurchlässigen Gewebematerial, insbesondere Gore-Tex -Material gefertigt. Alternativ hierzu, oder auch in Kombination mit dieser Maßnahme ist es auch möglich, die Abdeckeinrichtung aus einem Porösmaterial zu fertigen.

Die Abdeckeinrichtung ist in besonders vorteilhafter Weise aus einem flexiblen Material gefertigt, das unter Wirkung des Druckes im Maskeninneraum aufgespannt ist. Die Luftdurchlässigkeit des luftdurchlässigen Materials und die Fläche des hierdurch definierten Abschnitts ist vorzugsweise so gewählt, dass ein hinreichender Gasabstrom aus dem Maskeninneraum gewährleistet ist.

Die Abdeckeinrichtung oder die Dichtlippeneinrichtung kann mit einer Kopfbandeinrichtung gekoppelt sein. Die Kopfbandeinrichtung selbst kann ebenfalls zur Bereitstellung einer Gasabströmfläche herangezogen werden. Diese Gasabströmfläche kann durch eine schlauchartige Zone aus einem luftdurchlässigen Schlauchmaterial gebildet sein.

Die Dichtlippeneinrichtung ist vorzugsweise an die Abdeckeinrichtung angeklebt oder anvulkanisiert oder angespritzt. Es ist auch möglich, die Abdeckeinrichtung lösbar mit der Dichtlippeneinrichtung zu koppeln, oder die Dichtlippeneinrichtung integral mit der Abdeckeinrichtung auszubilden. Es ist möglich, die Abdeckeinrichtung durch Stützstrukturen aufzuspannen.

Die Abdeckeinrichtung kann auch einen Hartschalenkörper und einen mit diesem gekoppelten Auslassgewebeabschnitt aufweisen. Dieser Auslassgewebeabschnitt weist vorzugsweise eine Fläche von wenigstens 3.7 cm² auf.

Hierin wird auch beschrieben eine Kopfbandeinrichtung für eine Atemmaske, wobei die Kopfbandeinrichtung zumindest abschnittsweise aus einem luftdurchlässigen Material gefertigt ist und eine Leitungseinrichtung umfasst, die mit einem durch die Atemmaske definierten Maskeninnenraum in Verbindung steht, derart, dass ein Abstrom von unter Druck stehendem Atemgas aus dem Maskeninnenraum durch jenen kopfbandseitig vorgesehenen luftdurchlässigen Materialabschnitt erfolgen kann.

Die eingangs angegebene Aufgabe wird erfindungsgemäß gelöst durch eine Atemmaskenanordnung zur Anwendung einer CPAP-Therapie zur Behandlung schlafbezogener Atmunsstörungen mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehendem Maskeninnenraum, wobei im Zusammenspiel mit dem Gewölbekörper ein Luftführungspfad definiert ist, der sich von einem Atemgaseintrittsbereich zu einem Atemgasaustrittsbereich erstreckt, und sich zumindest abschnittsweise entlang der den Gewölbekörper begrenzenden Wandung erstreckt, dadurch gekennzeichnet, dass die Atemmaskenanordnung ein Einsatzelement aufweist und der Luftführungspfad durch das Einsatzelement begrenzt ist.

Dadurch wird es auf vorteilhafte Weise möglich, eine günstig zu reinigende Atemmaskenanordnung zu schaffen, die sich durch eine geringe Geräuschemission und ein geringes Totraumvolumen auszeichnet.

### Vorteilhafte Ausgestaltungen dieser Erfindung sind Gegenstand der entsprechenden Unteransprüche

Hierin werden auch folgende Punkte beschrieben.
1. Atemmaskenanordnung mit einer Dichtlippeneinrichtung zur Auflage auf der Gesichtsfläche eines Maskenanwenders, einer Abdeckeinrichtung die im Zusammenspiel mit der Dichtlippeneinrichtung einen Maskeninnenraum definiert, einer Atemgasleitungseinrichtung zur Zuleitung von Atemgas zu jenem von der Abdeckeinrichtung definiertem und mit der Nasen- und/oder Mundöffnung des Maskenanwenders in Verbindung stehendem Maskeninnenraum, wobei die Abdeckeinrichtung zumindest abschnittsweise als durchlässige Struktur ausgebildet ist.
2. Atemmaskenanordnung nach Punkt 1, dadurch gekennzeichnet, dass die Abdeckeinrichtung aus einem luftdurchlässigen Gewebematerial, insbesondere Gore-Tex -Material gefertigt ist.
3. Atemmaskenanordnung nach Punkt 1, dadurch gekennzeichnet, dass die Abdeckeinrichtung aus einem porösmaterial gefertigt ist.
4. Atemmaskenanordnung nach wenigstens einem der Punkte 1 bis 3, dadurch gekennzeichnet, dass die Abdeckeinrichtung aus einem flexiblen Material gefertigt ist, das unter Wirkung des Druckes im Maskeninneraum aufgespannt ist.
5. Atemmaskenanordnung nach wenigstens einem der Punkte 1 bis 4, dadurch gekennzeichnet, dass die Luftdurchlässigkeit des luftdurchlässigen Materials und die Fläche des hierdurch definierten Abschnitts so gewählt ist, dass ein hinreichender Gasabstrom aus dem Maskeninneraum gewährleistet ist.
6. Atemmaskenanordnung nach wenigstens einem der Punkte 1 bis 5, dadurch gekennzeichnet, dass die Abdeckeinrichtung mit einer Kopfbandeinrichtung gekoppelt ist.
7. Atemmaskenanordnung nach wenigstens einem der Punkte 1 bis 6, dadurch gekennzeichnet, dass die Kopfbandeinrichtung ebenfalls zur Bereitstellung einer Gasabströmfläche herangezogen wird.
8. Atemmaskenanordnung nach wenigstens einem der Punkte 1 bis 7, dadurch gekennzeichnet, dass die Dichtlippeneinrichtung and die Abdeckeinrichtung angeklebt oder anvulkanisiert oder angespritzt ist.
9. Atemmaskenanordnung nach wenigstens einem der Punkte 1 bis 7, dadurch gekennzeichnet, dass die Abdeckeinrichtung lösbar mit der Dichtlippeneinrichtung gekoppelt ist.
10. Atemmaskenanordnung nach wenigstens einem der Punkte 1 bis 9, dadurch gekennzeichnet, dass die Dichtlippeneinrichtung integral mit der Abdeckeinrichtung ausgebildet ist.
11. Atemmaskenanordnung nach wenigstens einem der Punkte 1 bis 10, dadurch gekennzeichnet, dass Stützstrukturenvorgesehen sind, zum Aufspannen der Abdeckeinrichtung.
12. Atemmaskenanordnung nach wenigstens einem der Punkte 1 bis 11, dadurch gekennzeichnet, dass die Stützwandstrukturen Teil einer Skelettstruktur bilden.
13. Atemmaskenanordnung nach wenigstens einem der Punkte 1 bis 12, dadurch gekennzeichnet, dass die Abdeckeinrichtung einen Hartschalenkörper und einen mit diesem gekoppelten Auslassgewebeabschnitt aufweist.
14. Atemmaskenanordnung nach Punkt 13, dadurch gekennzeichnet, dass der Auslassgewebeabschnitt eine Fläche von wenigstens 3,7 cm² aufweist.
15. Kopfbandeinrichtung für eine Atemmaske, wobei die Kopfbandeinrichtung zumindest abschnittsweise aus einem luftdurchlässigen Material gefertigt ist und eine Leitungseinrichtung umfasst, die mit einem durch die Atemmaske definierten Maskeninneraum in Verbindung steht, derart, dass ein Abstrom von unter Druck stehendem Atemgas aus dem Maskeninnenraum durch jenen kopfbandseitig vorgesehenen luftdurchlässigen Materialabschnitt erfolgen kann.

### Kurzbeschreibung der Zeichnungen

Weitere Einzelheiten und Merkmale der Erfindung ergeben sich aus der nachfolgenden Beschreibung in Verbindung mit der Zeichnung. Es zeigt:
**Fig. 1** eine Skizze zur Erläuterung einer ersten Ausführungsform einer hierin beschriebenen Atemmaske;
**Fig. 2** eine Skizze zur Erläuterung einer zweiten Ausführungsform einer Atemmaske;
**Fig. 3** eine Skizze zur Erläuterung eines Gewebematerialabschnitts;
**Fig. 4** eine Skizze zur Erläuterung der durch lockere Kette/Schuss-Bindung bei einem Gewebematerial erreichten Mikroporenstruktur.
**Fig. 5** eine Skizze zur Erläuterung eines aus einem Gewebematerial gebildeten Auslasseinsatzes für eine Maskenhartschale.
**Fig. 6** eine Skizze zur Erläuterung einer Ausführungsform der Erfindung;
**Fig. 7** eine Skizze zur Erläuterung eines bevorzugten Aufbaues einer Hartschale und eines zum Einsatz hierin vorgesehenen Einsatzelementes;
**Fig. 8a** eine Skizze zur Erläuterung einer bevorzugten Ausgestaltung eines Einsatzelementes der Applikationsstruktur der vorangehend gezeigten Atemmaskenanordnung;
**Fig. 8b** eine Skizze zur Erläuterung einer bevorzugten Innengestaltung einer Hartschale mit einem Aufnahmeabschnitt zur Aufnahme des Einsatzelementes nach Figur 8a.

### Ausführliche Beschreibung der Zeichnungen

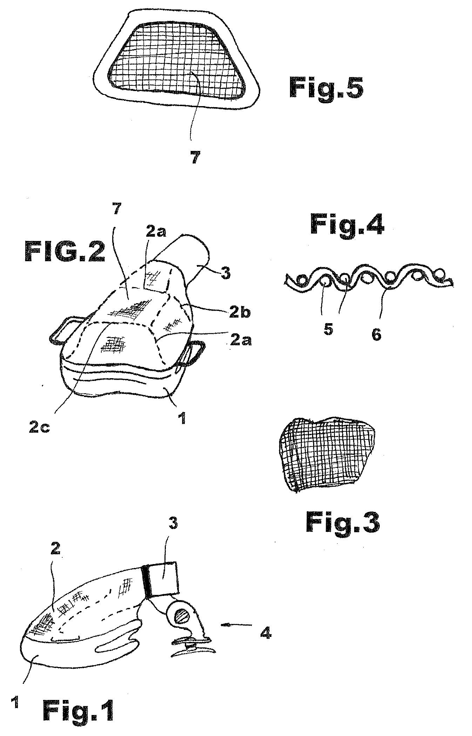

Die in Fig. 1 dargestellte Atemmaskenanordnung umfasst eine, aus einem Elastomermaterial, insbesondere Silikonkautschuk gefertigte Dichtlippeneinrichtung 1 und eine Abdeckeinrichtung 2. Die Dichtlippeneinrichtung 1 ist derart ausgebildet, dass diese eine, zur Aufnahme des Nasenbereichs eines Maskenanwenders vorgesehene Aufnahmeöffnung umsäumt und hierbei vorzugsweise den Nasenrücken sowie den Oberlippenbereich des Maskenanwenders überquert. Die Dichtlippeneinrichtung 1 hat hierbei eine im wesentlichen sattelförmige Silhouette.

Die Abdeckeinrichtung 2 ist derart ausgebildet, dass diese im Zusammenspiel mit der Dichtlippeneinrichtung 1 einen Maskeninnenraum definiert. Der Maskeninnenraum steht mit einer Atemgasleitungseinrichtung 3 zur Zuleitung von Atemgas zu jenem von der Abdeckeinrichtung definiertem und mit der Nasen- und/oder Mundöffnung des Maskenanwenders in Verbindung. Die Abdeckeinrichtung 2 ist zumindest abschnittsweise als luftdurchlässige Gewebestruktur ausgebildet. Die Atemgasleitungseinrichtung 3 bildet einen Anschlussstutzen zur Ankoppelung eines Atemgasschlauches. Die gezeigte Atemmaskenanordnung dient der Zufuhr von Atemgas auf einem Druckpegel der über dem Umgebungsdruck liegt. Die Abdeckeinrichtung 2 wird unter Wirkung des Druckes im Maskeninnenraum zwischen ihren randseitigen Anbindungsstellen, d.h. der Dichtlippeneinrichtung 2 und der Atemgasleitungseinrichtung 3 aufgespannt.

Figur 2 zeigt eine weitere Variante einer Atemmaske. Bei dieser Maske ist die Abdeckeinrichtung 2 durch Nahtabschnitte 2a, 2b, 2c, 2d abgesteppt ausgebildet. Der Verlauf der Nahtabschnitte, sowie die Gestalt der zwischen diesen Nahtabschnitten liegenden Gewebezonen, ist derart abgestimmt, dass die Abdeckeinrichtung unter Wirkung des Atemgasdruckes eine definierte Gestalt erreicht.

Es ist auch möglich, die Abdeckeinrichtung auf eine Rippenstruktur aufzusetzen, oder als Einsatzelement für eine Hartschale auszubilden.

Anstelle eines Gewebemateriales können auch Vlies- oder Filtermaterialien oder anderweitige Porösmaterialien, z.B. mikroperforierte Kunststofffolien Anwendung finden.

Figur 3 zeigt einen Ausschnitt einer aus einem Gewebematerial gefertigten Abdeckeinrichtung.

Figur 4 zeigt Kettfäden 5 und Schussfäden 6 des Gewebeabschnitts nach Figur 3. Zwischen den Kett- und Schussfäden sind Zwischenräume definiert durch welche CO2 befrachtetes Atemgas aus dem Maskeninnenraum entweichen kann.

Figur 5 zeigt ein Einsatzelement für eine Maskenhartschale. Das Einsatzelement umfasst eine Poröszone 7 die aus einem luftdurchlässigen Material gefertigt ist. Über diese Poröszone kann CO2 befrachtetes Atemgas aus dem Maskeninnenraum entweichen.

Die Befestigung der beschriebenen Atemmasken kann durch Kopfbandeinrichtungen erfolgen. Diese Kopfbandeinrichtungen können zur Ableitung von Atemgas herangezogen werden indem diese luftdurchlässige Zonen aufweisen, die über eine Leitungseinrichtung mit dem Maskeninnenraum in Verbindung stehen.

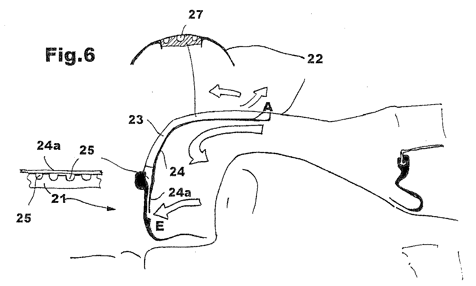

Die in Fig. 6 dargestellte Atemmaskenanordnung umfasst eine, aus einem Elastomermaterial, insbesondere Silikonkautschuk gefertigte Dichtlippeneinrichtung 21 und einen Gewölbekörper 22. Die Dichtlippeneinrichtung 21 ist derart ausgebildet, dass diese eine, zur Aufnahme des Nasenbereichs eines Maskenanwenders vorgesehene Aufnahmeöffnung umsäumt und hierbei vorzugsweise den Nasenrücken sowie den Oberlippenbereich des Maskenanwenders überquert. Die Dichtlippeneinrichtung 21 hat hierbei eine im wesentlichen sattelförmige Silhouette.

Der Gewölbekörper ist derart ausgebildet, dass dieser einen Luftführungspfad 23 definiert, der sich von einem Atemgaseintrittsbereich E zu einem Atemgasaustrittsbereich A erstreckt, und sich zumindest abschnittsweise entlang einer den Gewölbekörper 22 begrenzenden Wandung erstreckt.

Der Luftführungspfad 23 ist zum Maskeninnenbereich hin durch ein Einsatzelement 24 begrenzt. Weiterhin ist der Gewölbekörper 22 mit einer Fixiereinrichtung versehen, zur Anbringung des Einsatzelementes 24. An dem Einsatzelement 24 ist ein Abdeckabschnitt 24a ausgebildet, der auf einer Kanalrillenstruktur 25 aufliegt, die im Innenbereich der Dichtlippeneinrichtung 21 ausgebildet ist.

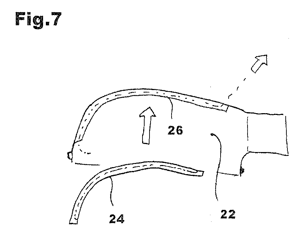

Wie aus Figur 7 ersichtlich, ist das Einsatzelement 24 im Innenbereich des Gewölbekörpers 22 anbringbar ist. Im Innenbereich des Gewölbekörpers 22 ist ein Aufnahmeabschnitt 26 ausgebildet ist, zur Aufnahme des Einsatzelementes 24.

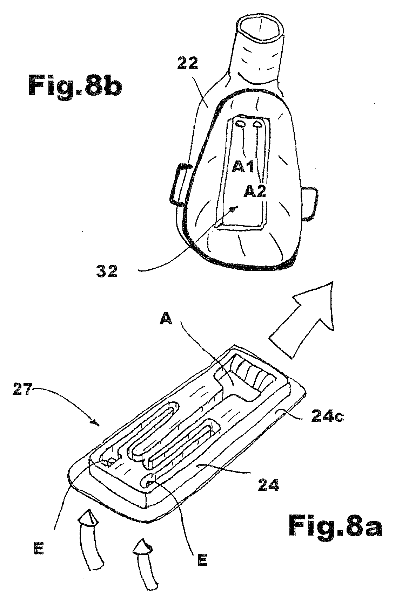

Wie aus Figur 8a ersichtlich sind in dem Einsatzelement 24 Kanalstrukturen 27 ausgebildet. Die Kanalstrukturen 27 sind derart abgestimmt, dass sich ein definierter Strömungswiderstand ergibt. Die Kanalstrukturen 27 erstrecken sich von einem Eintrittsbereich E zu einem Austrittsbereich A. Die Kanalabschnitte 27 sind im montierten Zustand von der Wandung des Gewölbekörpers 22 abgedeckt. Das Einsatzelement 24 ist aus einem Elastomermaterial gefertigt. In dem Einsatzelement 24 sind durch die Kanalabschnitte 27 Labyrinthstrukturen ausgebildet. Das Einsatzelement 24 ist durch Klemmung mit dem Gewölbekörper koppelbar. Zur zusätzlichen Abdichtung ist eine umlaufende Dichtlippe 24c an dem Einsatzelement 24 ausgebildet. Das hier gezeigte Einsatzelement ist in den in Figur 8b gezeigten Gewölbekörper 22 von innen einsetzbar.

Wie aus Figur 8b ersichtlich befinden sich in dem Gewölbekörper 22 Austrittsöffnungen A1, A2, die mit dem Austrittsbereich A des Einsatzelementes übereinkommen und eine Abströmung der verbrauchten Atemluft ermöglichen. Der Gewölbekörper 22 selbst ist bei diesem Ausführungsbeispiel aus einem thermoplastischen Kunststoffmaterial gefertigt. Der zur Aufnahme des Einsatzelementes 24 vorgesehene Aufnahmebereich 32 kann durch eine Vertiefung oder durch eine Umfangswandung definiert sein.

Es ist auch möglich, die Fixierung des Einsatzelementes durch Zapfen oder anderweitige Haltemittel zu bewerkstelligen.

Das Einsatzelement ist hier durch einen flach rechteckförmigen Grundkörper gebildet. Es ist auch möglich. das Einsatzelement anderweitig, insbesondere zylinderscheibenartig oder als Polygon-Prisma zu gestalten. Es ist auch möglich, das Einsatzelement so auszugestalten, dass sich in Abhängigkeit von seiner Position an dem Gewölbekörper bestimmte Drosselwirkungen ergeben.

Das Einsatzelement kann auch so gestaltet sein, dass sich in Abhängigkeit vom Innendruck unterschiedliche Strömungswiderstände, insbesondere durch Verformung der Kanalabschnitte 27 ergeben.

## Patentansprüche

1. Atemmaskenanordnung zur Anwendung einer CPAP-Therapie zur Behandlung schlafbezogener Atmungsstörungen mit einem Gewölbekörper (22), einer Dichtlippeneinrichtung (1, 21) zur Auflage auf der Gesichtsfläche eines Maskenanwenders, einer Atemgasleitungseinrichtung (3) zur Zuleitung von Atemgas zu einem von dem Gewölbekörper (22) begrenzten und mit der Nasen- und/oder Mundöffnung des Maskenanwenders in Verbindung stehenden Maskeninnenraum, wobei im Zusammenspiel mit dem Gewölbekörper (22) ein Luftführungspfad (23) definiert ist, der sich von einem Atemgaseintrittsbereich (E) zu einem Atemgasaustrittsbereich (A) erstreckt, und sich zumindest abschnittsweise entlang einer den Gewölbekörper (22) begrenzenden Wandung erstreckt, **dadurch gekennzeichnet, dass** die Atemmaskenanordnung ein Einsatzelement (24) aufweist und der Luftführungspfad (23) durch das Einsatzelement (24) begrenzt ist.

2. Atemmaskenanordnung nach Anspruch 1, **dadurch gekennzeichnet, dass** der Gewölbekörper (22) mit einer Fixiereinrichtung versehen ist, zur Anbringung des Einsatzelementes (24).

3. Atemmaskenanordnung nach wenigstens einem der Ansprüche 1 bis 2. **dadurch gekennzeichnet, dass** im Innenbereich des Gewölbekörpers (22) ein Aufnahmeabschnitt (26) ausgebildet ist, zur Aufnahme des Einsatzelementes (24).

4. Atemmaskenanordnung nach wenigstens einem der Ansprüche 1 bis 3, **dadurch gekennzeichnet, dass** in dem Einsatzelement (24) Kanalstrukturen (27) ausgebildet sind.

5. Atemmaskenanordnung nach Anspruch 4, **dadurch gekennzeichnet, dass** die Kanalstrukturen (27) derart abgestimmt sind, dass sich ein definierter Strömungswiderstand ergibt.

6. Atemmaskenanordnung nach wenigstens einem der Ansprüche 4 bis 5, **dadurch gekennzeichnet, dass** die Kanalstrukturen (27) von der Wandung des Gewölbekörpers (22) abgedeckt sind.

7. Atemmaskenanordnung nach wenigstens einem der Ansprüche 1 bis 6, **dadurch gekennzeichnet, dass** der Gewölbekörper (22) aus einem Elastomermaterial gefertigt ist.

8. Atemmaskenanordnung nach wenigstens einem der Ansprüche 1 bis 7, **dadurch gekennzeichnet, dass** der Gewölbekörper (22) integral mit der Dichtlippeneinrichtung (1, 21) ausgebildet ist.

9. Atemmaskenanordnung nach wenigstens einem der Ansprüche 1 bis 8, **dadurch gekennzeichnet, dass** der Gewölbekörper (22) mit Öffnungen (A1, A2) versehen ist, zur Ableitung von CO2 befrachtetem Atemgas.

10. Atemmaskenanordnung nach wenigstens einem der Ansprüche 1 bis 9, **dadurch gekennzeichnet, dass** das Einsatzelement (24) aus einem Elastomermaterial gefertigt ist.

11. Atemmaskenanordnung nach wenigstens einem der Ansprüche 1 bis 10, **dadurch gekennzeichnet, dass** in dem Einsatzelement (24) Labyrinthstrukturen ausgebildet sind.

12. Atemmaskenanordnung nach wenigstens einem der Ansprüche 1 bis 11, **dadurch gekennzeichnet, dass** das Einsatzelement (24) durch Klemmung mit dem Gewölbekörper (22) gekoppelt ist.

13. Atemmaskenanordnung nach einem der Ansprüche 1 bis 12, wobei das Einsatzelement (24) im Innenbereich des Gewölbekörpers (22) anbringbar ist.

## Claims

1. A respiratory mask arrangement to be used in CPAP therapy for the treatment of sleep-related breathing disorders having an arched member (22), a sealing lip device (1, 21) for resting on the facial surface of a mask user, a respiratory gas conduit unit (3) for delivering respiratory gas to a mask interior being defined by the arched member (22) and being in communication with the nostril and/or the oral opening of the mask user, wherein in cooperation with the arched member (22) an air guide path (23) is defined, the air guide path extending from a respiratory gas inlet area (E) to a respiratory gas outlet area (A) and extending at least in some portions along a wall that defines the arched member (22),
**characterized in that**
the respiratory mask arrangement comprises an insert element (24) and the air guide path (23) is defined by the insert member (24).

2. The respiratory mask arrangement according to claim 1, **characterized in that** the arched member (22) is provided with a fixation device for installing the insert element (24).

3. The respiratory mask arrangement according to at least one of claims 1 to 2, **characterized in that** a receiving portion (26) for receiving the insert element (24) is formed in the inner region of the arched member (22).

4. The respiratory mask arrangement according to at least one of claims 1 to 3, **characterized in that** conduit structures (27) are formed within the insert element (24).

5. The respiratory mask arrangement according to claim 4, **characterized in that** the conduit structures (27) are adapted such that a defined flow resistance is obtained.

6. The respiratory mask assembly according to at least one of claims 4 to 5, **characterized in that** the conduit structures (27) are covered by the wall of the arched member (22).

7. The respiratory mask arrangement according to at least one of claims 1 to 6, **characterized in that** the arched member (22) is made from an elastomer material.

8. The respiratory mask arrangement according to at least one of claims 1 to 7, characterized that the arched member (22) is formed integrally with the sealing lip device (1, 21).

9. The respiratory mask arrangement according to at least one of claims 1 to 8, **characterized in that** the arched member (22) is provided with openings (A1, A2) for discharging CO₂-laden respiratory gas.

10. The respiratory mask arrangement according to at least one of claims 1 to 9, **characterized in that** the insert element (24) is made from an elastomer material.

11. The respiratory mask arrangement according to at least one of claims 1 to 10, **characterized in that** labyrinth structures are formed in the insert element (24).

12. The respiratory mask arrangement according to at least one of claims 1 to 11, **characterized in that** the insert element (24) is coupled to the arched member (22) by clamping.

13. The respiratory mask assembly according to any one of claims 1 to 12, wherein the insert element (24) can be mounted in the interior region of the arched member (22).

## Revendications

1. Unité de masque respiratoire pour l'application d'une thérapie CPAP de traitement de troubles respiratoires du sommeil, avec un corps bombé (22), un agencement de lèvres d'étanchéité (1, 21) destiné à être appliqué sur la surface du visage d'un utilisateur du masque, un dispositif de conduit de gaz respiratoire (3) pour l'amenée de gaz respiratoire vers un intérieur de masque délimité par le corps bombé (22) et relié aux orifices nasaux et/ou à la bouche de l'utilisateur du masque, un chemin de guidage d'air (23) étant défini en interaction avec le corps bombé (22), lequel s'étend d'une zone d'entrée de gaz respiratoire (E) à une zone de sortie de gaz respiratoire (A), et au moins en partie le long d'une paroi délimitant le corps bombé (22), **caractérisé en ce que** ladite unité de masque respiratoire comporte un élément d'insertion (24) et **en ce que** le chemin de guidage d'air (23) est délimité par ledit élément d'insertion (24).

2. Unité de masque respiratoire selon la revendication 1, **caractérisé en ce que** le corps bombé (22) est pourvu d'un dispositif de fixation pour la mise en place de l'élément d'insertion (24).

3. Unité de masque respiratoire selon au moins une des revendications 1 et 2, **caractérisé en ce qu'**une section de réception (26) est formée à l'intérieur du corps bombé (22) pour la réception de l'élément d'insertion (24).

4. Unité de masque respiratoire selon au moins une des revendications 1 à 3, **caractérisé en ce que** des structures de canal (27) sont formées dans l'élément d'insertion (24).

5. Unité de masque respiratoire selon la revendication 4, **caractérisé en ce que** les structures de canal (27) sont ajustées de manière à obtenir une résistance définie à l'écoulement.

6. Unité de masque respiratoire selon au moins une des revendications 4 et 5, **caractérisé en ce que** les structures de canal (27) sont couvertes par la paroi du corps bombé (22).

7. Unité de masque respiratoire selon au moins une des revendications 1 à 6, **caractérisé en ce que** le corps bombé (22) est fabriqué dans un matériau élastomère.

8. Unité de masque respiratoire selon au moins une des revendications 1 à 7, **caractérisé en ce que** le corps bombé (22) est intégré à l'agencement de lèvres d'étanchéité (1, 21).

9. Unité de masque respiratoire selon au moins une des revendications 1 à 8, **caractérisé en ce que** le corps bombé (22) est pourvu d'orifices (A1, A2) pour l'évacuation du gaz respiratoire chargé en CO2.

10. Unité de masque respiratoire selon au moins une des revendications 1 à 9, **caractérisé en ce que** l'élément d'insertion (24) est fabriqué dans un matériau élastomère.

11. Unité de masque respiratoire selon au moins une des revendications 1 à 10, **caractérisé en ce que** des structures en labyrinthe sont formées dans l'élément d'insertion (24).

12. Unité de masque respiratoire selon au moins une des revendications 1 à 11, **caractérisé en ce que** l'élément d'insertion (24) est accouplé par enclenchement au corps bombé (22).

13. Unité de masque respiratoire selon une des revendications 1 à 12, où l'élément d'insertion (24) peut être mis en place à l'intérieur du corps bombé (22).
